# EUROPÄISCHE PATENTSCHRIFT

(11) **EP 2 964 087 B1**
(45) Veröffentlichungstag und Bekanntmachung des Hinweises auf die Patenterteilung: **28.09.2016**
(21) Anmeldenummer: 14827161.2
(22) Anmeldetag: 27.11.2014
(51) Int. Cl.: A61B 5/055, A61B 5/00

(54) **PATIENTENLIEGE FÜR DIE NMR-MAMMOGRAPHIE**
PATIENT SUPPORT FOR NMR MAMMOGRAPHY
LIT D'EXAMEN POUR IRM DU SEIN

(30) Priorität: 29.11.2013 DE 102013113276
(43) Veröffentlichungstag der Anmeldung: 13.01.2016
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2014/100417
(87) Internationale Veröffentlichungsnummer: WO 2015/078452

(56) Entgegenhaltungen:
- DE-A1-102007 006 855
- DE-A1-102009 056 176
- DE-U1-202006 004 127
- US-A1- 2005 228 267
- US-A1- 2007 223 652
- US-A1- 2007 250 047

## Beschreibung

Die Erfindung betrifft eine Patientenliege für die NMR- Mammographie mit einem Probanden sowie zwei Öffnungen, die zum Durchfädeln von jeweils einer Brust bestimmt sind und in deren unmittelbarer Nähe Spulen vorhanden sind.

Das Mammakarzinom als einer der gefährlichsten bösartigen Tumore ist aufgrund seiner weiten Verbreitung die häufigste Todesursache bei Frauen. Den Vorsorgeuntersuchungen zu Zwecken der Früherkennung kommt deshalb höchste Bedeutung zu. Als bildgebende Untersuchungsverfahren werden in der Röntgenmammographie Röntgenstrahlen, in anderen Untersuchungsverfahren Ultraschall (Sonographie) und in letzter Zeit immer häufiger die Kernspintomographieverfahren eingesetzt. Bei letzteren hat sich erwiesen, dass die Sensitivität, d.h. die Empfindlichkeit des Nachweises von Krebsgeweben, höher ist als bei der Untersuchung mit Röntgenstrahlen oder Ultraschall. Das Untersuchungsprinzip besteht im Wesentlichen darin, dass in einem konstanten Magnetfeld befindliche Protonen durch ein elektromagnetisches Wechselfeld von außen eine Energiezufuhr erhalten, die zu einer Anregung der Spinenergie führt. Mit Relaxation, d.h. nach Rückkehr in seinen Ausgangszustand wird von dem Spin eine Radiowelle emittiert, die durch geeignete Detektoren erfasst werden. Je größer die Anzahl der Protonen ist, wie sie insbesondere bei wasserhaltigem Gewebe in erhöhtem Maße vorkommen, umso höher ist die emittierte und gemessene Signaldichte. Hierdurch lassen sich anatomische Anomalien erkennen und beurteilen. Bei Brustuntersuchungen werden Kontrastmittel appliziert, die sich in besonderer Weise dort anreichern, wo ein erhöhter Blutfluss herrscht und die zu einer wesentlichen Verbesserung der Bildaufnahmequalität und deren Aussagekraft führen. Die Weiterentwicklung und Verbesserung derartiger Geräte zur Brustmammographie sind Gegenstand der nachfolgenden Erfindung.

US 2007/250047 und US 2005/228267 offenbaren Patientenliegen für die NMR-Mammographie. US 2007/223652 und DE 10 2009 056176 zeigen Kompressionsbänder für die Mammographie.

Die Durchführung einer NMR- Mammographie erfolgt in der Weise, dass die Patientin bäuchlings auf eine Patientenliege verbracht wird, dort die Brüste jeweils in eine Öffnung durchgefädelt und unterhalb der Liege untersucht werden. Zu diesem Zweck werden in unmittelbarer Nähe des zu untersuchenden Gewebes die Empfangsspulen für die emittierten Radiowellen angebracht.

Die vorliegende Erfindung hat sich die Weiterentwicklung dieser Geräte zur Brustmammographie zur Aufgabe gemacht und zielt darauf ab, die Untersuchung in möglichst kurzer Zeit durchführen zu können und eine optimale Bildqualität zu erhalten.

Gelöst wird diese Aufgabe erfindungsgemäß dadurch, dass im Bereich einer Öffnung unterhalb der Liege ein Band angebracht ist, das von der Außenseite der Liege bzw. des Probanden medial nach innen zu geführt ist, dort die Brust cranial bzw. caudal umgibt, um dann von der medialen Seite her an der Brust anliegend zur caudalen bzw. cranialen Anlage an die Brust zu verlaufen, wobei die Enden des Bandes lateral nach außen geführt und dort benachbart zueinander verlaufen.

Die Grundlagen der erfindungsgemäßen Vorschläge bestehen in der Erkenntnis, dass eine allseitige Fixierung der Brust sicherstellt, dass es zu keinen Relativbewegungen des Messobjektes während der Dauer der Aufnahme und demzufolge zu Unschärfen in der Bildqualität kommt und zum anderen, dass die Anzahl der aufzunehmenden Schichten, unabhängig davon, ob sie in transversaler oder sagittaler Richtung verlaufen, aufgrund der Kompressionen der Brust zu einem Minimum werden. Je geringer die Anzahl der aufzunehmenden Schichten zur Abbildung des gesamten Objektes ist, umso schneller lässt sich die Aufnahme durchführen. Wird der aufzunehmende Gewebebereich in seinen Abmessungen stark verkleinert, kann die Aufnahme auch schneller durchgeführt werden als es bei der Aufnahme von Brüsten größerer Abmessungen der Fall wäre.

Die vorgeschlagene Lösung besteht darin, dass ein Band eingesetzt wird, das von der Außenseite medial nach innen zu geführt wird, dort die Brust cranial bzw. caudal umgibt, um dann von der medialen Seite her an der Brust anliegend zur caudalen bzw. cranialen Anlage an die Brust zu kommen. Das Band wird dann lateral nach außen geführt und endet dann am oder in der Nähe des Ausgangspunktes des Bandes. Die Fixierung mit Hilfe des Bandes geschieht in der Weise, dass das Band in axialer Richtung und bezogen auf die Körperebene in lateraler Richtung nach außen in axialer Richtung gespannt wird und hierdurch die allseitige Kompression der Brust erfolgt. Das Band bildet eine nahezu geschlossene Schleife, welche um die Brust gelegt und zur Fixierung zusammengezogen wird.
In der Praxis ist bedeutungslos, ob beide Enden des Bandes in axialer Richtung eine Zugbeanspruchung erfahren, oder ob das eine Ende fixiert und nur das andere Ende zugbelastet wird. Die Spannung komprimiert die Brust über den gesamten Umfang und in radialer Richtung nach innen und führt zu einer Fixierung für die Dauer der Durchführung der NMR-Aufnahme. Die Fixierung erfolgt durch einen einzigen durchgehenden Zug in axialer Richtung am Ende des Bandes.

Im Rahmen der Erfindung ist hierbei unerheblich, auf welche Art und Weise die Zugkräfte ausgeübt werden, ob es sich also um manuelle oder einen motorischen Spannvorgang handelt.
Ein weiterer wesentlicher Vorteil ist auch darin zu sehen, dass im fixierten Zustand Gewebeproben dadurch entnehmbar sind, dass mit einer Biopsienadel die Bänder durchgestochen werden. Der fixierte Zustand erlaubt eine besonders präzise und zielgerichtete Entnahme des jeweiligen Gewebes.

Für die konkrete bauliche Gestaltung und Umsetzung des erfindungsgemäßen Gedankens bestehen im Rahmen der Erfindung verschiedene Möglichkeiten. Als besonders bevorzugt wird eine Lösung vorgeschlagen, bei der ein Andruckblock eingesetzt wird, der in lateraler Richtung außerhalb der Brust angeordnet wird und welcher der endwärtigen Aufnahme des ausgehenden und auch des zurückgeführten Bandes dient. Hierzu sind zwei, etwa senkrecht zur Körperebene verlaufende Schlitze im Abstand zueinander vorgesehen, welche dem Einfädeln der beiden Enden des Bandes dienen, wobei die Enden noch nach außen zu überstehen. Die Bänder bilden in medialer Richtung vom Andruckblock ausgehend und zwar im Wesentlichen konzentrisch zur Brustöffnung eine Schlinge, in der Weise, dass beim Einführen der Brust zunächst ein Abstand zwischen Brust und Band gegeben ist. Wird nun in axialer Richtung ein Zug auf eines oder beiden der Enden und zwar von lateral außerhalb des Andrucksblockes ausgeübt, führt dies dazu, dass sich die Schlinge soweit zusammenzieht und die Brust selbst unter Einschluss des Andruckblockes vollständig umschließt. Der axiale Zug wird solange fortgesetzt, bis ein Widerstand die flächige Anlage am Brustgewebe anzeigt und die gewünschte Kompression aufgebaut hat. Grundsätzlich ist denkbar, dass nicht nur die Gurte axial unter Spannung gesetzt werden, sondern dass auch zusätzlich der Andruckblock in medialer Richtung verschoben wird. Das Ergebnis ist, dass mit einer einzigen Spannbewegung und damit sehr rasch eine allseitig wirksame Fixierung der Brust in kürzester Zeit vorgenommen werden kann.

Für die Führung des Bandes sind in zweckmäßigen Ausgestaltungen verschiedene Maßnahmen denkbar. Nachdem das Band in der Ausgangsposition die Öffnung für das Einfädeln der Brust freigeben muss, andernfalls wäre das Einführen behindert oder zumindest erschwert, empfiehlt sich, den Gurt im Randbereich der Öffnung oder in radialer Richtung nach außen zu vorübergehend zu sichern. Zu diesem Zweck besitzt das Band auf seiner Außenfläche einen Klettverschluss, der mit einer entsprechenden Gegenfläche zusammenarbeitet. Wird nun Zug auf das Band in axialer Richtung ausgeübt, löst sich der Klettverschluss und das Band kann zum Mittelpunkt der Öffnung, also zum Zentrum der Brust hin bewegt werden und hierdurch die gewünschte Fixierung vornehmen.

Eine weitere Möglichkeit besteht darin, dass dem Andruckblock gegenüber ein Steg befindlich ist, durch den das Band gefädelt wurde. Durch diese Maßnahme wird die Öffnung zunächst vom Band freigehalten, sodass auch hier das Einfädeln der Brust erleichtert wird und bei Zug wird dann das am Steg festliegende Band den Andruckblock in Richtung auf den Steg zu bewegen und auf diese Weise in die Fixierposition überzugehen.

In einer baulichen Alternative von großer Bedeutung wird vorgeschlagen, das Band in Form eines Polygons und hier insbesondere eines Rechteckes in einer ebenfalls in einer zur Körperebene parallelen Ebene zu führen und in den Eckpunkten jeweils eine Umlenkrolle anzubringen, die die Bänder einen Winkel von 90 Grad und mehr beschreiben lassen und der zwei Anpressrollen zugeordnet sind, die dafür Sorge tragen, dass das Band an der Umlenkrolle sowohl eingehend als auch ausgehend von der Anpressrolle in Richtung der (lokalen) Winkelhalbierenden gepresst wird und somit im Ergebnis die Anpressrollen in etwa aufeinander zu bewegt werden. Die Anpressrollen stellen sicher, dass das Band über den gesamten Umfang in Körperkontakt tritt und die Umlenkrolle selbst beanstandet bleibt, sodass es zu keinen Berührungen kommen kann. Die Anpressrollen haben zur Folge, dass das Band nahezu über den gesamten Umfang der Umlenkrolle geführt wird. Die Anpassung und Fixierung der Brust geschieht in der Weise, dass die aus den zwei Anpressrollen und der Umlenkrolle bestehenden Eckpunkte über einen Schlitten in 2 Richtungen, z.B. mit Hilfe von Kreuzschlitten nach Art eines kartesischen Koordinatensystems, verfahrbar sind und sich so weit aufeinander zu bewegen, bis das Band allseitig an der Brust zur Anlage kommt. Die Bewegung der Eckpunkte wird dadurch ausgelöst, dass auf das Band ein Zug in axialer Richtung ausgeübt wird, sodass die Eckpunkte entgegen einer Rückstellkraft "zusammengezogen" werden. Auch bei dieser baulichen Lösung ist unerheblich, ob das eine Ende des Gurtes raumfest fixiert ist und nur das andere Ende zugbelastet werden kann, oder ob beide Enden des Gurtes, die dann unmittelbar aneinander liegen, gleichzeitig oder unabhängig voneinander zugbelastet werden.
Auch bei dieser Vorrichtung erhält man eine rasche Fixierung der Brust mit den angestrebten Vorteilen.

In einer zweckmäßigen Ausgestaltung ist vorgeschlagen, sowohl in der Fixiereinheit und/oder in den Bändern mit Spulen zu bestücken, die medial oder lateral einschieb- oder einsteckbar sind.

Bei den Bändern selbst kann es sich um sterile Produkte handeln, die es erlauben, Gewebeproben zu entnehmen. Auch ist denkbar, dass in den Bändern ein Gitter mit Öffnungen, in der Fachsprache als Grid bezeichnet, eingebracht werden kann.

Die bisherigen Betrachtungen haben sich darauf beschränkt, eine Kompression und Fixierung der Brust in einer Ebene parallel zur Körperebene vorzunehmen. In einer Weiterbildung empfiehlt sich deshalb, eine Spule von unten her, d.h. senkrecht zur dieser Ebene und damit in Richtung auf die Brustwarze zu, heranzufahren und anzupressen, um auch von dieser Seite her eine Kompression und die gewünschte Minimierung des zu untersuchenden Brustvolumens zu erreichen. Durch das mit Spulen bestückte Fixierelement erfolgt eine Bewegung senkrecht zur vorbeschriebenen Ebene, sodass man im Ergebnis eine dreidimensionale Fixierung der Brust erhält. Durch die weitere Spule wird zusätzlich eine erhebliche Verbesserung der Bildqualität erzielbar.

Für die Probanden wird häufig als unangenehm empfunden, dass sich das Körpergewicht nahezu ausschließlich über das Brustbein (Sternum) auf der Unterlage abstützt. Es empfiehlt sich deshalb, Stützen in einer Richtung senkrecht zur Patientenliege und außerhalb des Sternums nach oben fahrbar anzubringen und den Körper auch hierdurch zu unterstützen. Auf diese Weise erfolgt eine Verteilung des Kraftflusses und eine Verminderung der punktuellen Druckbelastung, was der Proband als angenehm empfindet.

Weitere Einzelheiten und Merkmale der Erfindung werden im folgenden Beschreibungsteil anhand eines Ausführungsbeispiels näher erläutert. Es zeigen in schematischer Darstellung:
- **Figur 1**: Fixiervorrichtung mit einem Andruckblock
- **Figur 2**: Fixiervorrichtung mit einem Band, das in Form eines Rechtecks geführt ist

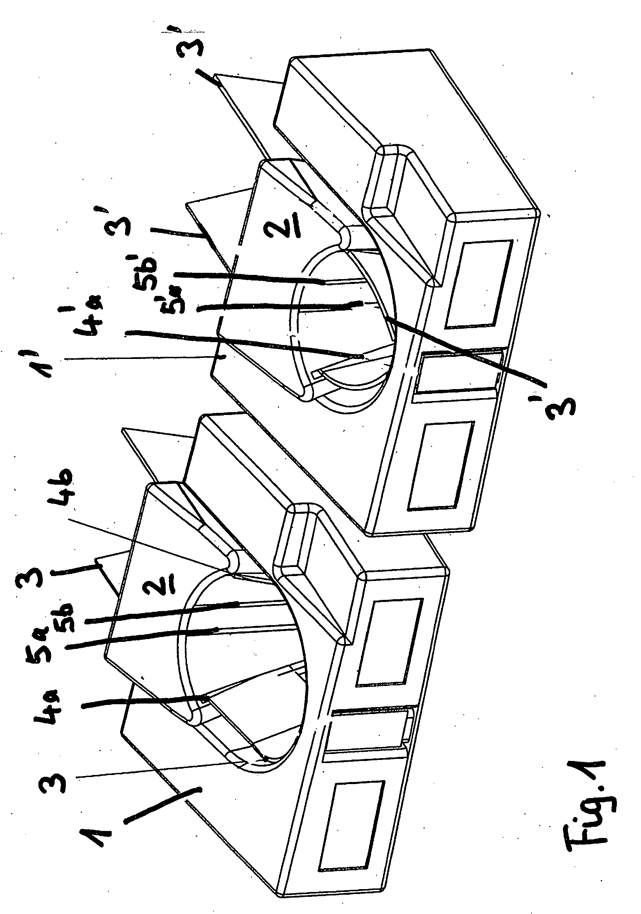

**Figur 1** zeigt einen Andruckblock 2, der in einer Fixiervorrichtung 1 verschiebbar untergebracht ist, die ihrerseits unterhalb des Patienten in einer (nicht dargestellten) Patentenliege integriert wird. Zur Verdeutlichung sind lediglich die Fixiereinrichtung 1 in prinzipienhaft gehaltener Darstellung wiedergegeben, wobei durch unmittelbaren Vergleich der beiden nebeneinander gezeigten verschiedenen Positionen zweier Fixiervorrichtungen 1 die Erkennung der kinematischen Verhältnisse deutlich wird. Dabei zeigt die linke Fixiervorrichtung 1 diejenige Phase, in der eine Brust noch nicht eingeführt ist, hingegen die rechte Fixiereinrichtung 1', diejenige, in der die (ebenfalls nicht gezeigte) Brust fixiert ist. Der Andruckblock 2 ist relativ zur Fixiervorrichtung 1 in deren Ebene verschiebbar, wobei die Verschieberichtung im eingebauten Zustand in medialer (oder lateral beim Öffnen) Richtung erfolgt. Die Fixiervorrichtung 1 beschreibt eine von dem Rand der Fixiervorrichtung 1 ausgehende Nut, die im Randbereich über den Andruckblock 2 verschlossen ist, wobei die Form von dieser Nut und der Stirnseite des Andruckblocks 2 so geformt sind, dass eine im Wesentlichen kreisförmige und durchgehende Öffnung verbleibt. In diese Öffnung wird später die Brust mit ihrer Mittelachse senkrecht zur Oberfläche der Fixiervorrichtung und somit auch senkrecht zur Körperebene des Patienten eingeführt.
Zur Fixierung wird das Band 3 eingesetzt, welches von der Außenfläche des Andruckblocks 2 ausgeht, einen korrespondierenden Schlitz 4 durchgreift und auf der Innenseite aus dem Andruckblock 2 austritt, daran anschließend etwa in Form eines Halbkreises geführt ist und wieder zurück zum Andruckblock 2 gelangt, wo über einen weiteren Schlitz 4' das Band 3 nach außen geführt ist. Dabei verläuft das Band im Wesentlichen spiegelsymmetrisch zu einer Mittelebene, die in etwa parallel zum Verlauf des Bandes 3 orientiert ist. Die beiden Enden des Bandes 3 stehen nach außen über, um einen Zugriff auf das Ende des Bandes 3 zu ermöglichen und einen axialen Zug auf das Band 3 ausüben zu können. Im Bereich des Halbkreises ist das Band 3 entlang der Innenfläche der Fixiereinrichtung 1 geführt. Eine lösbare Befestigung des Bandes 3, die nicht eingezeichnet ist, gewährleistet das Freihalten des gesamten Querschnitts, sodass sich das Durchfädeln der Brust möglichst einfach gestaltet.
Der Andruckblock 2 zeigt auf seiner Innenfläche benachbart zu den Schlitzen 4 und 4' etwa in der Mittelebene zwei weitere Schlitze 5, 5', die parallel zueinander verlaufen und welche die Möglichkeit eröffnen, dass die Enden des Bands 3 dort anstelle in den Schlitzen 4, 4' eingefädelt werden können. Das Ergebnis ist dann eine Einengung der durch Fixiervorrichtung 1 und Andruckblock 2 beschriebenen lichten Weite, was die Phase des Einfädelns und der Fixierung selbst verkürzt.

In der rechts neben der soeben beschriebenen gezeigten Vorrichtung 1' ist jene Phase gezeigt, in welcher die (nicht dargestellte) Brust fixiert ist. Eine Beschreibung der einzelnen Teile ist bereits im Zusammenhang mit der linken Darstellung erfolgt und kann zu Vermeidung von Wiederholungen unterbleiben. Die in der rechten Figur wiedergegebenen Bezugszeichen stimmen mit denjenigen der linken Darstellung überein, wurden jedoch zur Unterscheidung mit Apostroph versehen.
Der Unterschied demgegenüber besteht jedoch darin, dass der Andruckblock 2 nach innen zu verschoben ist, was dadurch erfolgt, dass ein axialer Zug auf das eine oder beide Enden des Bandes 3 ausgeübt wird. Hierdurch wird auch die Verbindung des Bandes 3 mit der in der Öffnung verlaufenden Stirnfläche zumindest teilweise gelöst. Die durch das Band 3 gebildete Schlinge zieht sich soweit zusammen, bis es zur allseitigen Anlage durch das Band 3 und den Andruckblock 2 an der Brust kommt. Damit ist die Fixierung der Brust beendet und das Erstellen der Aufnahmen kann beginnen.

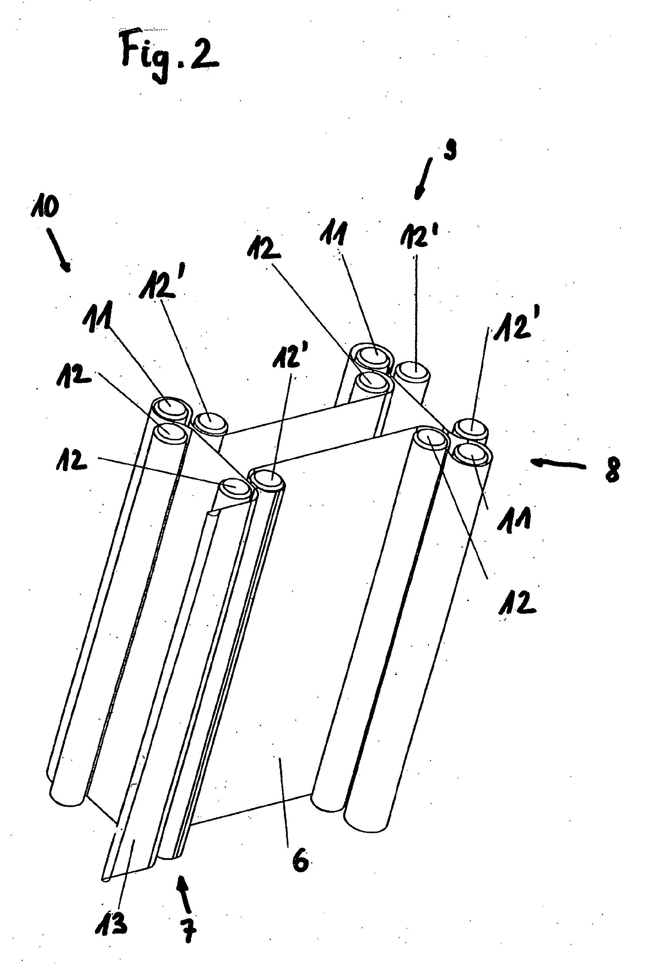

In **Figur 2** ist eine alternative Realisierung der erfindungsgemäßen Fixiervorrichtung gezeigt. In der dort gezeigten Darstellung wird das Band 6 zwischen vier Eckpunkten 7, 8, 9, 10 derart geführt, dass das Band 6 bei Draufsicht, d. h. bei Blickrichtung in der Ebene des Bandes 6 von seinem Grundsatz her ein Rechteck beschreibt. Die einander gegenüberliegend befindlichen Flächen des Bandes 6 sind hierbei parallel zueinander ausgerichtet. Mit Ausnahme des Eckpunkts 7, sind die Eckpunkte 8 - 10 in ihrem Grundsatz her von gleichem Aufbau, d. h. sie bestehen aus einer Umlenkrolle 11 und jeweils zwei Anpressrollen 12. Die Führung geschieht in der Weise, dass in den Eckpunkten die Umlenkrollen 11 angebracht sind und das der Umlenkrolle 11 zugeführte Band 6 über eine der beiden Anpressrollen 12 angepresst wird und das wegführende Band 6 über die zweite Anpressrolle 12' ebenfalls angepresst wird und zwar in der Weise, dass das Band 6 die Umlenkrolle 11 nahezu vollständig umschließt. Jede der beiden Anpressrollen 12 (bzw. 12') drücken das Band 6 in Richtung auf die gegenüber liegende Anpressrolle 12' (bzw. 12). Jener Bereich des Bandes 6, der sich zwischen den Eckpunkten 7 - 10, genauer deren Anpressrollen 12, 12' befindet, wird zur Fixierung der Brust genutzt.
Von den Eckpunkten 8 - 10 unterscheidet sich der Eckpunkt 7 dadurch, dass anstelle der Umlenkrolle das Band 6 mit zumindest dem einen Ende nach außen geführt ist und die Möglichkeit eröffnet, in axialer Richtung Zug auf das Band 6 auszuüben. Das gegenüberliegende Ende des Bands 6 ist an der zu dieser Umlenkrolle 11 gehörigen Anpressrolle 12' fixiert.
In der Zeichnung nicht zu erkennen ist, dass die die Eckpunkte 7 - 10 bildende Anordnung entgegen einer Rückstellkraft in einer Ebene senkrecht zum Band 6 verfahrbar sind. Bei einer rechteckförmigen Führung des Bandes 6, wie gezeigt, können hierzu Kreuzschlitten genutzt werden.
Die Benutzung geschieht in der Weise, dass die Eckpunkte 7- 10 soweit auseinander gefahren werden, bis die zu untersuchende Brust zwischen das aus den Bändern 6 gebildeten Rechteck eingefädelt werden kann. Anschließend wird durch Zug auf das Ende 13 des Bandes 6 eine Kraft ausgeübt, die bewirkt, dass alle Eckpunkte 7 - 10 aufeinander zu bewegt werden, sodass die Brust eine Einschnürung erfährt und eine entsprechende Gegenkraft aufbaut. Auf diese Weise wird die Brust bleibend fixiert und die Aufnahmen können vorgenommen werden.
Den gezeigten Ausführungsbeispielen gemeinsam ist, dass ein Band 3 eingesetzt wird, welches die Brust schlingenförmig umgreift und bei deren Zusammenziehen in axialer Richtung eine Fixierung erfolgt.

### Bezugszeichenliste

- 1: Fixiervorrichtung
- 2: Andruckblock
- 3: Band
- 4a, 4b: (äußerer) Schlitz
- 5a, 5b: (innerer) Schlitz
- 6: Band
- 7 - 10: Eckpunkt
- 11: Umlenkrolle
- 12, 12': Anpressrolle
- 13: Bandende

## Patentansprüche

1. Patientenliege für die NMR- Mammographie mit einem Probanden sowie zwei Öffnungen, die zum Durchfädeln von jeweils einer Brust bestimmt sind und in deren unmittelbarer Nähe Spulen vorhanden sind, **dadurch gekennzeichnet, dass** im Bereich einer Öffnung unterhalb der Liege ein Band (3) angebracht ist, das von der Außenseite der Liege bzw. des Probanden medial nach innen zu geführt ist, dort die Brust cranial bzw. caudal umgibt, um dann von der medialen Seite her an der Brust anliegend zur caudalen bzw. cranialen Anlage an die Brust zu verlaufen, wobei die Enden des Bandes (13) lateral nach außen geführt und dort benachbart zueinander verlaufen.

2. Vorrichtung nach Anspruch 1, **dadurch gekennzeichnet, dass** ein Andruckblock (2) eingesetzt wird, der in lateraler Richtung außerhalb der Brust angeordnet wird und welcher der endwärtigen Aufnahme des ausgehenden und auch des zurückgeführten Bandes (3) dient und der relativ zur Vorrichtung (1) bewegbar ist.

3. Vorrichtung nach Anspruch 2, **dadurch gekennzeichnet, dass** im Andruckblock (2) wenigstens zwei, etwa senkrecht zur Körperebene verlaufende Schlitze im Abstand zueinander vorgesehen sind, in welche der beiden Enden des Bandes (13) eingefädelt sind und die Enden noch nach außen zu überstehen.

4. Vorrichtung nach einem der vorhergehenden Ansprüche, **dadurch gekennzeichnet, dass** entweder beide Enden des Bandes (3) in axialer Richtung eine Zugbeanspruchung erfahren, oder ob das eine Ende fixiert und nur das andere Ende zugbelastet ist.

5. Vorrichtung nach einem der vorhergehenden Ansprüche, **dadurch gekennzeichnet, dass** eine manuelle oder einen motorischen Spannvorrichtung vorhanden ist.

6. Vorrichtung nach einem der vorhergehenden Ansprüche, **dadurch gekennzeichnet, dass** das Band (3) auf seiner Außenfläche einen Klettverschluss aufweist, der mit einer entsprechenden Gegenfläche zusammenarbeitet.

7. Vorrichtung nach einem Anspruch 2, **dadurch gekennzeichnet, dass** dem Andruckblock (2) gegenüber ein Steg befindlich ist, durch den das Band (3) gefädelt und der Andruckblock (2) in Richtung auf den Steg zu bewegbar ist.

8. Vorrichtung nach Anspruch 1, **dadurch gekennzeichnet, dass** das Band in Form eines Polygons in einer zur Körperebene parallelen Ebene geführt ist und in den Eckpunkten jeweils eine Umlenkrolle (11) angebracht ist, die das Band (3) umlenken und denen jeweils zwei Anpressrollen (12, 12') zugeordnet sind, die sowohl das zugeführte als auch ausgehende Band (3) an der Umlenkrolle (11) in Richtung der lokalen Winkelhalbierenden presst und die aus den zwei Anpressrollen (12, 12') und der Umlenkrolle (11) bestehenden Eckpunkte (7 - 10) über einen Schlitten in zwei Richtungen z. B. nach Art eines kartesischen Koordinatensystems, entgegen einer Rückstellkraft verfahrbar sind, wobei ein Eckpunkt (7) nur aus zwei Anpressrollen (12, 12') besteht und dort die Enden des Bandes (13) nach außen geführt sind.

9. Vorrichtung nach einem der vorhergehenden Ansprüche, **dadurch gekennzeichnet, dass** die Vorrichtung (1) und/oder die Bändern (3, 6) mit Spulen bestückt sind, die medial oder lateral einschieb- oder einsteckbar sind.

10. Vorrichtung nach einem der vorhergehenden Ansprüche, **dadurch gekennzeichnet, dass** die Bänder (3, 6) steril sind und/oder Öffnungen für Gitter aufweisen.

11. Vorrichtung nach einem der vorhergehenden Ansprüche, **dadurch gekennzeichnet, dass** eine Spule von unten her senkrecht zur der durch das Band (3) definierte Ebene und in Richtung auf die Brustwarze zu heranfahrbar angebracht ist.

12. Vorrichtung nach einem der vorhergehenden Ansprüche, **dadurch gekennzeichnet, dass dadurch** Stützen in Richtung senkrecht zur Patientenliege und außerhalb des Sternums nach oben zu fahrbar angebracht sind.

## Claims

1. Patient table for NMR mammography comprising a test subject and two openings, which are intended for inserting a breast in each case, coils being present in the immediate vicinity thereof, **characterised in that** in the region of an opening, below the table, a band (3) is attached, which is guided medially inwardly from the outside of the table or of the test subject, here surrounds the breast cranially or caudally, and then runs from the medial side, bearing against the breast for caudal or cranial contact with the breast, the ends of the band (13) being guided laterally outwardly, where they run adjacent to one another

2. Device according to claim 1, **characterised in that** a pressure block (2) is used, which is disposed in a lateral direction outside the breast and which serves for the reception of the outgoing and also of the returned band (3) at its ends and which can be moved relative to the device (1).

3. Device according to claim 2, characterised that, in the pressure block (2), at least two slits, which run approximately perpendicular to the plane of the body, are provided at a distance from one another, in which the two ends of the band (13) are threaded in and the ends still project outwardly.

4. Device according to one of the preceding claims, **characterised in that** either both ends of the band (3) in the axial direction undergo tensile stressing, or one end is fixed and only the other end is subjected to tensile stress.

5. Device according to one of the preceding claims, **characterised in that** a manual or motor-driven tensioning device is present.

6. Device according to one of the preceding claims, **characterised in that** the band (3) has a hook-and-loop fastening on its outer face, which cooperates with a corresponding counter surface.

7. Device according to claim 2, **characterised in that** the pressure block (2) is located opposite a bridge, through which the band (3) is threaded and the pressure block (2) is movable towards the bridge.

8. Device according to claim 1, **characterised in that** the band is guided in the form of a polygon in a plane that is parallel to the plane of the body, and at the corner points a deflector roller (11) in each case is mounted, which deflect the band (3), and to which two pressure rollers (12, 12') in each case are assigned, which press the both the infed and outgoing band (3) against the deflector roller (11) in the direction of the local angle-bisector, and the corner points (7-10) consisting of the two pressure rollers (12, 12') and the deflector roller (11) are traversable, against a restoring force, via a slide in two directions, for example in the manner of a Cartesian coordinate system, a corner point (7) consisting only of two pressure rollers (12, 12') and here the ends of the band (13) being guided outwardly.

9. Device according to one of the preceding claims, **characterised in that** the device (1) and/or the bands (3, 6) are equipped with coils, which can be pushed in or plugged in medially or laterally.

10. Device according to one of the preceding claims, **characterised in that** the bands (3, 6) are sterile and/or have openings for grids.

11. Device according to one of the preceding claims, **characterised in that** a coil is mounted from below perpendicular to the plane defined by the band (3) such that it can be traversed through said plane towards the nipple.

12. Device according to one of the preceding claims, **characterised in that** supports are thereby mounted that are traversable upwardly, perpendicular to the patient table and outside the sternum.

## Revendications

1. Table d'examen médical destinée à la mammographie RMN, avec un proband ainsi que deux ouvertures qui sont chacune destinées à enfiler un sein et à proximité immédiate desquelles se trouvent des bobines, **caractérisée par le fait qu'**une bande (3) est disposée dans la zone d'une ouverture en dessous de la table, laquelle bande peut être amenée vers l'intérieur depuis le côté extérieur de la table ou du proband médial, y entoure le sein de façon crâniale ou caudale, pour suivre ensuite, depuis le côté médial en reposant sur le sein, un parcours le long du sein vers l'installation médiale ou crâniale, sachant que les extrémités de la bande (13) sont menées latéralement vers l'extérieur pour finalement se rejoindre.

2. Dispositif selon la revendication 1, **caractérisé par le fait qu'**on utilise un bloc de pression (2), qui est disposé dans le sens latéral à l'extérieur du sein et qui sert à la réception, à l'extrémité, de la bande (3) sortante et aussi de la bande ramenée et qui peut être déplacé par rapport au dispositif (1).

3. Dispositif selon la revendication 2, **caractérisé par le fait que** dans le bloc de pression (2) sont prévues au moins deux fentes suivant un parcours à peu près vertical par rapport au plan du corps et à une certaine distance l'une d'entre elle, dans lesquelles sont enfilées les deux extrémités de la bande (13) qui dépassent encore vers l'extérieur.

4. Dispositif selon une des revendications précédentes, **caractérisé par le fait que** les deux extrémités de la bande (3) sont soumises à une sollicitation par traction dans le sens axial, à moins qu'une extrémité ne soit fixée, l'autre extrémité étant soumise à une traction.

5. Dispositif selon une des revendications précédentes, **caractérisé par le fait qu'**il existe un dispositif de serrage manuel ou motorisé.

6. Dispositif selon une des revendications précédentes, **caractérisé par le fait que** la bande (3) présente sur sa surface extérieure une fermeture velcro qui fonctionne avec une surface opposée correspondante.

7. Dispositif selon la revendication 2, **caractérisé par le fait qu'**il y a, en face du bloc de pression (2), une traverse à travers laquelle la bande (3) est enfilée, le bloc de pression (2) pouvant être déplacé dans un sens vers cette traverse.

8. Dispositif selon la revendication 1, **caractérisé par le fait que** la bande est menée sous la forme d'un polygone dans un niveau parallèle au plan, un rouleau de renvoi (11) étant disposé dans chacun des angles, lesquels rouleaux de renvoi (11) renvoient la bande (3), deux rouleaux de pression (12, 12') étant attribués à chacun d'entre eux, lesquels pressent à la fois la bande (3) amenée et la bande (3) sortante sur le rouleau de renvoi (11) dans le sens des demi-angles locaux, les angles (7 - 10) consistant en les deux rouleaux de pression (12, 12') et le rouleau de renvoi (11) pouvant être déplacés, selon un système de coordonnées cartésiennes, par l'intermédiaire d'un chariot dans deux directions contre une force de rappel, sachant qu'un angle (7) consiste seulement en deux rouleaux de pression (12, 12') et que les extrémités de la bande (13) y sont menées vers l'extérieur.

9. Dispositif selon une des revendications précédentes, **caractérisé par le fait que** le dispositif (1) et/ou les bandes (3, 6) sont équipés de bobines qui peuvent être insérées ou enfichées dans le sens médial ou latéral.

10. Dispositif selon une des revendications précédentes, **caractérisé par le fait que** les bandes (3, 6) sont stériles et/ou présentent des ouvertures pour des grilles.

11. Dispositif selon une des revendications précédentes, **caractérisé par le fait qu'**une bobine peut être amenée par déplacement à la verticale depuis le bas vers le niveau défini par la bande (3) et en direction du mamelon.

12. Dispositif selon une des revendications précédentes, **caractérisé par le fait que** des appuis sont ainsi disposés, en pouvant être déplacés, dans le sens vertical par rapport à la table d'examen médical et vers le haut à l'extérieur du sternum.
